# EUROPEAN PATENT APPLICATION

(11) **EP 2 623 092 A1**
(43) Date of publication of application: **07.08.2013**
(21) Application number: 11829269.7
(22) Date of filing: 29.09.2011
(51) Int. Cl.: A61K 8/58, A61Q 19/00

(54) **COSMETIC COMPOSITION**

(30) Priority: 01.10.2010 JP 2010224114
(71) Applicant: l'Foret Co., Ltd., Yokohama-shi, Kanagawa 247-8630 (JP); Shirai, Hirofusa, Ueda-shi, Nagano 386-0407 (JP); Daiwabo Neu Co., Ltd., Osaka-shi, Osaka 541-0056 (JP); Daiwabo Holdings Co., Ltd., Osaka-shi, Osaka 541-0056 (JP)
(72) Inventor: SHIRAI Hirofusa, Ueda-shi Nagano 386-0407 (JP); IWAMA Tomiko, Yokohama-shi Kanagawa 247-8630 (JP); TSUIKI Hisanaga, Osaka-shi Osaka 541-0056 (JP)
(74) Representative: Grünecker, Kinkeldey, Stockmair & Schwanhäusser
(86) International application number: PCT/JP2011/072365
(87) International publication number: WO 2012/043716

(57) **Abstract**

A highly moisturizing cosmetic composition having firmness/elasticity-improving effect, wrinkle-improving effect, and texture-improving effect can be provided by blending a metal phthalocyanine derivative or salt thereof in a cosmetic material. For the metal phthalocyanine derivative, iron phthalocyanine tetracarboxylic acid is particularly favorable.

## Description

### Technical Field

The present invention relates to a cosmetic composition.

### Prior Art

Representative changes to human skin caused by aging include wrinkles and sagging, which are primary concerns for women. However, few effective treatments for wrinkling and sagging of the skin have been available to date.

Today, a drop in skin elasticity is considered a cause of wrinkling and sagging of human skin, and this drop in elasticity has to do with such factors as aging, drying, oxidization, and sunlight (UV rays), and the like. Specifically from the histological phenomenon, collagen, elastin, glycosaminoglycan and other extra-cellular matrix components in the dermis are changing. A drop in the skin's moisture-retaining property is also considered a cause.
Metal phthalocyanine derivatives are artificially synthesized, water-soluble, blue coloring agents that are used widely as fiber dyes and pigments. They are also very safe. Metal phthalocyanine derivatives are also known to provide an anti-bacterial effect, deodorizing effect, allergen decomposition effect, itchiness-suppressing effect (refer to Patent Documents I to 3), and the like. Accordingly, anti-allergenic underwear and masks using fibers dyed with metal phthalocyanine derivatives are being examined/implemented (Patent Document 4). Phthalocyanine-based coloring agents are also used in some makeup cosmetic materials (Patent Document 5). However, metal phthalocyanine derivatives are used in these products only for the purpose of coloring and not moisturizing the skin and preventing skin aging. On the other hand, Patent Document 6 discloses a cosmetic material decomposing peroxide lipids, containing a sulfonated phthalocyanine or salt metal thereof.

In addition, collagen peptide-containing cosmetic compositions offering a moisturizing effect are already known. For example, a technology has been shown to obtain a peptide composition by decomposing the collagen component with cysteine protease, and this technology is claimed useful for cosmetic materials, etc. (refer to Patent Document 7, for example). However, in some cases a satisfying effect is not always obtained even when such component is used, and therefore a cosmetic material offering anti-skin-aging effect and moisturizing effect, which can be applied to various conditions and concerns, is needed.

### Prior Art Documents

### Patent Documents

[Patent Document 1] Japanese Patent Laid-open No. 2001-303437
[Patent Document 2] Japanese Patent Laid-open No. Hei 06-32177
[Patent Document 3] Japanese Patent Laid-open No. 2005-144219
[Patent Document 4] Japanese Patent Laid-open No. 2004-321409
[Patent Document 5] Japanese Patent Laid-open No. 2003-335976
[Patent Document 6] Japanese Patent Laid-open No. Hei 03-50285
[Patent Document 7] Japanese Patent Laid-open No. 2006-342107

### Summary of the Invention

### Problems to Be Solved by the Invention

The object of the present invention is to provide a highly moisturizing cosmetic composition having a firmness/elasticity-improving effect, wrinkle-improving effect, and texture-improving effect.

### Means for Solving the Problems

While studying metal phthalocyanine having a function to decompose allergenic substances, the inventors found that a cosmetic material containing this metal phthalocyanine would provide a skin-moisturizing effect, improve wrinkles of the skin, and also improve the skin texture, and they finally completed the present invention.
In other words, the present invention has the following constitutions:
1. A cosmetic composition characterized by containing a metal phthalocyanine derivative expressed by General Formula [I]. 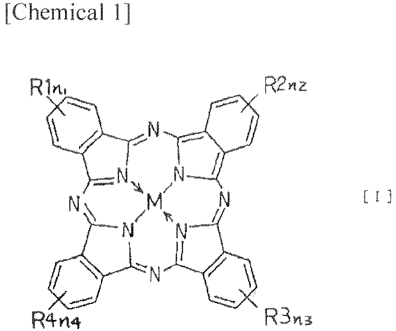 (In Formula [I], M represents a metal selected from the group that includes Fe, Co, Mn, Ti, V, Ni, Cu, Zn, Mo, W and Os, R1 to R4 are each a carboxylic group, and n1 to n4 are each an integer of 0 to 4 satisfying "1 ≤ n1 + n2 + n3 + n4 ≤ 8.")
2. A cosmetic composition according to 1, characterized by containing, as an effective ingredient, a metal phthalocyanine derivative per Formula [I] above where R1 to R4 are each a carboxylic group and n1 to n4 all take the same value of 1 or 2.
3. A cosmetic composition according to 1 or 2, characterized by containing, as an effective ingredient, a metal phthalocyanine derivative per Formula [I] above where M is Fe.
4. A cosmetic composition according to any one of 1 to 3, wherein the cosmetic composition is used for skincare cosmetic materials.
5. A cosmetic composition according to any one of I to 4, wherein the metal phthalocyanine derivative is an iron phthalocyanine tetracarboxylic acid or salt thereof.
6. A cosmetic composition according to any one of I to 4, wherein the metal phthalocyanine derivative is an iron phthalocyanine octacarboxylic acid or salt thereof.
7. A cosmetic composition characterized by containing a metal phthalocyanine derivative expressed by Formula [I] above as well as a sequestering agent or a component having a sequestering effect.
8. A cosmetic composition according to 7, wherein the sequestering agent is at least one type of substance selected from the group that includes sodium EDTA-2, sodium EDTA-3, sodium EDTA-4, etidronic acid, sodium etidronate 4, hydroxy ethane diphosphonic acid solution, and citric acid, while the component having the sequestering effect is at least one type of substance selected from the group that includes succinic acid, phytic acid, ascorbic acid, gluconic acid, phosphoric acid, sodium polyphosphate, and sodium metaphosphate.
9. A cosmetic composition according to 7 or 8, characterized by containing, as an effective ingredient, a metal phthalocyanine derivative per Formula [I] above where R1 to R4 are each a carboxylic group and n1 to n4 all take the same value of I or 2.
10. A cosmetic composition according to any one of 7 to 9, characterized by containing, as an effective ingredient, a metal phthalocyanine derivative per Formula [I] above where M is Fe, Co or Mn.
11. A cosmetic composition according to any one of 7 to 10, wherein the cosmetic composition is used for skincare cosmetic materials.
12. A cosmetic composition according to any one of 7 to 11, wherein the metal phthalocyanine derivative is an iron phthalocyanine tetracarboxylic acid or salt thereof.
13. A cosmetic composition according to any one of 7 to 11, wherein the metal phthalocyanine derivative is an iron phthalocyanine octacarboxylic acid or salt thereof.

### Effects of the Invention

The cosmetic composition proposed by the present invention can improve fine lines, firmness/elasticity, texture, dull complexion, and moisture-retaining property of the skin, and as a skincare cosmetic material it can improve skin aging as a whole. The cosmetic composition proposed by the present invention can also keep the color of phthalocyanine stable for a long period of time.

### Brief Description of the Drawings

[Fig. 1] illustrates a graph relating to overall evaluation.
[Fig. 2] illustrates a graph relating to evaluation of individual items.

### Best Mode for Carrying Out the Invention

The cosmetic composition proposed by the present invention is characterized in that it contains metal phthalocyanine carboxylic acid. Accordingly, this cosmetic composition offers excellent moisturizing effect and anti-skin-aging effect.
Here, the term "anti-skin-aging" as it is used in connection with the present invention means preventing changes that occur in the skin tissues due to aging, exposure to UV rays, etc., and examples include improvement in wrinkles , texture, firmness, and dull complexion, and maintenance/improvement of moisturized feel and elasticity of the skin.

The cosmetic composition proposed by the present invention may be a water-based composition, oil-based composition, or emulsion composition.
Examples of the water-based composition include skin lotion, beauty essence, water-based gel, and the like, while examples of the oil-based composition include cleansing oil and oil-based gel, and the like. Examples of the emulsion composition include cream, skin milk and sunscreen lotion, and the like, where the types of emulsion include O/W, W/O and multilayer emulsion (W/O/W, O/W/O), etc.

The following explains metal phthalocyanine derivatives according to the present invention.

In other words, the cosmetic composition proposed by the present invention contains, as an active ingredient, a metal phthalocyanine derivative expressed by Formula [I] below.

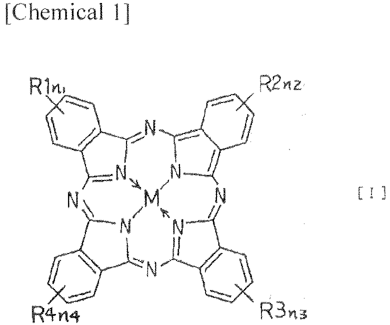

Note that, in Formula [I], n 1 to n4 are each an integer of 0 to 4 satisfying "1 ≤ n1 + n2 + n3 + n4 ≤ 8," where preferably n1 to n4 all take the same value of 1 or 2, while M is a metal selected from the group that includes Fe, Co, Mn, Ti, V, Ni, Cu, Zn, Mo, W and Os. Preferably the substitution groups R1 to R4 are each a carboxylic group. Since metal phthalocyanine carboxylic acid or salt thereof catalyzes and breaks down aldehyde compounds that are said to have adverse effects on the skin, it is estimated to be effective on fine lines, firmness/elasticity, texture, dull complexion, and moisturizing of the skin. In addition, metal phthalocyanine carboxylic acid or salt thereof is estimated to have higher hydrophilicity and therefore offer greater moisturizing effect than sulfonated phthalocyanine.

In particular, a metal phthalocyanine derivative whose substitution groups R1 to R4 are each a carboxylic group and n1 to n4 are each 1 in Formula [I], or specifically metal phthalocyanine tetracarboxylic acid or salt thereof expressed by Formula [II] below, is particularly effective.

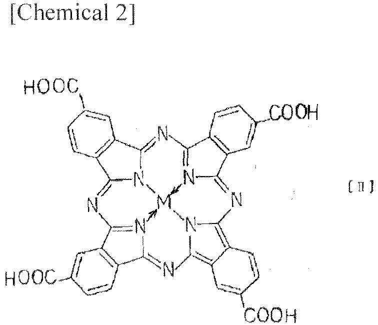

In addition, a metal phthalocyanine derivative whose substitution groups R1 to R4 are each a carboxylic group and n1 to n4 are each 2 in Formula [I], or specifically metal phthalocyanine octacarboxylic acid or salt thereof expressed by Formula [III] below, is also particularly effective.

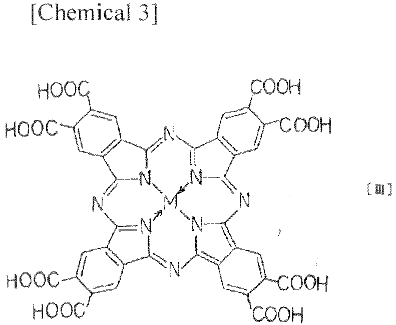

The central metal M in Formulas [I] to [III] is as defined earlier, but it is preferably Fe, Co or Mn of high-spin type or more preferably Fe.

Among the metal phthalocyanine derivative structures, iron phthalocyanine tetracarboxylic acid or salt thereof is the most preferable. Since iron phthalocyanine carboxylic acid or salt thereof is less irritating to the skin, it can be used favorably as a cosmetic component. On the other hand, metal phthalocyanine sulfonate or salt thereof (sulfonated phthalocyanine) irritates the skin because sulfonic acid or salt thereof is a strong acid or strong acid salt, and therefore it cannot be used favorably as a cosmetic component.

Examples of the salt of metal phthalocyanine carboxylic acid include salt with an inorganic base or salt with an organic base, among others. Favorable examples of the salt with an inorganic base include, among others, sodium salt, potassium salt and other alkaline metal salts; calcium salt, magnesium salt and other alkaline earth metal salts; and copper (II) salt and ammonium salt. Favorable examples of the salt with an organic base include, among others, salts with trimethyl amine, triethyl amine, pyridine, picoline, ethanol amine, diethanol amine, triethanol amine, dicyclohexyl amine, etc.

The metal phthalocyanine derivative in Formula [I] may be a commercial product or manufactured according to a known method. For example, it can be manufactured according to the method described in "Futaroshianin - Kagaku to Kinou-" (Phthalocyanine - Chemistry and Function -) (Hirofusa Shirai, Nagao Kobayashi; Industrial Publishing & Consulting; issued February 28 1997). For example, iron phthalocyanine tetracarboxylic acid can be obtained as follows. Specifically, it can be obtained after trimellitic anhydride, urea, ammonium molybdate, and ferric chloride anhydrous are added to nitrobenzene and the mixture is stirred and heated/refluxed to obtain precipitate, to which alkali is added to cause hydrolysis, and then acid is added to turn the hydrolyzate acidic.
Cobalt phthalocyanine octacarboxylic acid, which is also a favorable example, can also be manufactured using a similar method by using pyromellitic anhydride instead of trimellitic anhydride, and cobaltic chloride instead of ferric chloride anhydrous, among the aforementioned materials of iron phthalocyanine tetracarboxylic acid.
Iron phthalocyanine octacarboxylic acid, which is also a favorable example, can also be manufactured using a similar method by using pyromellitic anhydride instead of trimellitic anhydride among the aforementioned materials of iron phthalocyanine tetracarboxylic acid.
Note that manufacturing methods of metal phthalocyanine derivatives are disclosed in detail in several documents including Japanese Patent Laid-open No. Sho 56-63355.
Among the metal phthalocyanine derivative structures, iron phthalocyanine tetracarboxylic acid or iron phthalocyanine octacarboxylic acid is the most preferable, as mentioned above.

With the cosmetic composition proposed by the present invention, the content of metal phthalocyanine derivative compound is preferably in a range of 0.00001 to 20 percent by mass, or more preferably in a range of 0.00005 to 10 percent by mass, or yet more preferably in a range of 0.0001 to 2 percent by mass, relative to the total mass of the composition.
Containing the metal phthalocyanine derivative compound by 0.00001 percent by mass or more is preferable from the viewpoint of moisturizing property. However, a high concentration of metal phthalocyanine derivative compound causes the compound to assume a dark blue to black color, which does not present functional problems at all, but is not desirable in terms of esthetic characteristics of the finished product which is a cosmetic material, and therefore the concentration of metal phthalocyanine derivative compound is preferably kept to 2 percent by mass or less. From this viewpoint, for example, iron phthalocyanine tetracarboxylic acid or iron phthalocyanine octacarboxylic acid, if blended, preferably has a concentration of 0.005 to 0.1 percent by mass. At this concentration, the composition will assume a beautiful navy blue or blue color.

### [Surfactant Contained in Water Dispersion]

Any surfactant can be used in the water dispersion obtained under the present invention as long as it is a water-soluble surfactant that dissolves in a water-based medium, for example, a nonionic surfactant with a HLB of 10 or more, or preferably 12 or more, is preferable. If the HLB is less than 10, sufficient emulsifying power may not be achieved. On the other hand, the HLB is preferably 16 or less in order to achieve stable emulsification.

Any surfactant can be used under the present invention, but a nonionic surfactant is preferred. Examples of the nonionic surfactant include glycerin fatty acid ester, organic acid monoglyceride, polyglycerin fatty acid ester, propylene glycol fatty acid ester, polyglycerin condensed ricinoleic acid ester, sorbitan fatty acid ester, and sucrose fatty acid ester, and the like. Polyglycerin fatty acid ester, sorbitan fatty acid ester, and sucrose fatty acid ester are more preferred. Note that the above surfactant need not have been highly refined by means of distillation, etc., and may be a reaction mixture.

The polyglycerin fatty acid ester used under the present invention is an ester of polyglycerin with an average degree of polymerization of 2 or more, or preferably 6 to 15, or more preferably 8 to 10 on one hand, and fatty acid with 8 to 18 carbons such as caprylic acid, capric acid, lauric acid, myristic acid, palmitic acid, stearic acid, oleic acid, or linoleic acid on the other. Preferable examples of such polyglycerin fatty acid ester include, among others, hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, and decaglycerin monolauric acid ester. These polyglycerin fatty acid esters may be used alone or mixed together. Examples of commercial products include, among others: NIKKOL DGMS, NIKKOL DGMO-CV, NIKKOL DGMO-90V, NIKKOL DGDO, NIKKOL DGMIS, NIKKOL DGTIS, NIKKOL Tetraglyn 1-SV, NIKKOL Tetraglyn 1-O, NIKKOL Tetraglyn 3-S, NIKKOL Tetraglyn 5-S, NIKKOL Tetraglyn 5-O, NIKKOL Hexaglyn 1-L, NIKKOL Hexaglyn I-M, NIKKOL Hexaglyn 1-SV, NIKKOL Hexaglyn 1-O, NIKKOL Hexaglyn 3-S, NIKKOL Hexaglyn 4-B, NIKKOL Hexaglyn 5-S, NIKKOL Hexaglyn 5-O, NIKKOL Hexaglyn PR-15, NIKKOL Decaglyn 1-L, NIKKOL Decaglyn I-M, NIKKOL Decaglyn 1-SV, NIKKOL Decaglyn 1-50SV, NIKKOL Decaglyn 1-ISV, NIKKOL Decaglyn 1-O, NIKKOL Decaglyn 1-OV, NIKKOL Decaglyn 1-LN, NIKKOL Decaglyn 2-SV, NIKKOL Decaglyn 2-ISV, NIKKOL Decaglyn 3-SV, NIKKOL Decaglyn 3-OV, NIKKOL Decaglyn 5-SV, NIKKOL Decaglyn 5-HS, NIKKOL Decaglyn 5-IS, NIKKOL Decaglyn 5-OV, NIKKOL Decaglyn 5-O-R, NIKKOL Decaglyn 7-S, NIKKOL Decaglyn 7-O, NIKKOL Decaglyn 10-SV, NIKKOL Decaglyn 10-IS, NIKKOL Decaglyn 10-OV, NIKKOL Decaglyn 10-MAC and NIKKOL Decaglyn PR-20 by Nikko Chemicals; Ryoto Polyglycerol Ester L-10D, L-7D, M-10D, M-7D, P-8D, S-28D, S-24D, SWA-20D, SWA-15D, SWA-10D, O-50D, O-15D, B-100D, B-70D and ER-60D by Mitsubishi-Kagaku Foods; Sunsoft Q-17UL, Sunsoft Q-14S and Sunsoft A-141C by Taiyo Chemical; and Poem DO-100 and Poem J-0021 by Riken Vitamin.

The sorbitan fatty acid ester used under the present invention has fatty acid with preferably 8 or more carbons, or more preferably 12 or more carbons. Preferable examples of such sorbitan fatty acid ester include, among others, sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate. These sorbitan fatty acid esters may be used alone or mixed together. Examples of commercial products include, among others: NIKKOL SL-10, SP-10V, SS-10V, SS-10MV, SS-15V, SS-30V, SI-10RV, SI-15RV, SO-10V, SO-15MV, SO-15V, SO-30V, SO-10R, SO-15R, SO-30R and SO-15EX by Nikko Chemicals; and Sorgen 30V, 40V, 50V, 90 and 1 10 by Dai-ichi Kogyo Seiyaku.

The sucrose fatty acid ester used under the present invention has fatty acid with preferably 12 or more carbons, or more preferably 12 to 20 carbons. Preferable examples of such sucrose fatty acid ester include, among others, sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester. Under the present invention, these sucrose fatty acid esters may be used alone or mixed together. Examples of commercial products include, among others: Ryoto Sugar Ester S-070, S-170, S-270, S-370, S-370F, S-570, S-770, S-970, S-1170, S-1170F, S-1570, S-1670, P-070, P-170, P-1570, P-1670, M-1695, O-170, O-1570, OWA-1570, L-195, L-595, L-1695, LWA-1570, B-370, B-370F, ER-190, ER-290 and POS-135 by Mitsubishi-Kagaku Foods; and DK Ester SS, F160, F140, F110, F90, F70, F50, F-A50, F-20W, F-10, F-A10E, Cosmelike B-30, S-10, S-50, S-70, S-110, S-160, S-190, SA-10, SA-50, P-10, P-160, M-160, L-10, L-50, L-160, L-150A, L-160A, R-10, R-20, O-10 and O-150 by Dai-ichi Kogyo Seiyaku.

Any such surfactant is added preferably by 0.1 to 50 percent by mass, or more preferably by 0.5 to 20 percent by mass, or yet more preferably by I to 15 percent by mass, relative to the water dispersion.
Adding the surfactant by 0.1 percent by mass or more is desirable as it achieves emulsion of finer particles and also ensures sufficient stability of emulsion, while adding the surfactant by 50 percent or less by mass is desirable as it adjusts foaming of emulsifier to an appropriate range.

### [Size of Emulsion Particles in Water Dispersion]

The size of emulsion particles in water dispersion is 200 nm or less, or preferably 150 nm or less, or more preferably 100 nm or less, by volume-average particle size (median size) from the viewpoint of transparency.
The particle size varies depending on such factors as the types and quantities of added components, emulsifying conditions (shear force, temperature, pressure) used for the manufacturing method, quantities of additives, ratio of oil phase and water phase, and quantity of surfactant, but there should not be any problems in practical settings as long as the particle sizes specified by the present invention are followed.

The particle size can be measured using any commercial particle size distribution analyzer, etc. Known methods for measuring the size distribution of emulsion include optical microscope method, confocus laser microscope method, electron microscope method, interatomic-force microscope method, static light scattering method, laser diffraction method, dynamic light scattering method, centrifugal sedimentation method, electric pulse measurement method, chromatography method, supersonic attenuation method, etc., and systems supporting each of these principles are commercially available.
Given the particle size ranges under the present invention and also to facilitate measurement, preferably the dynamic light scattering method is used for measurement of emulsion particle size under the present invention. Commercially available measuring systems using dynamic light scattering include, among others, Nanotrac UPA (Nikkiso), dynamic light scattering size distribution measuring system LB-550 (Horiba) and rich size analyzer FPAR-1000 (Otsuka Electronics). Any measurement temperature may be selected as long as it is normally used for measurement of particle size, but preferably the temperature is 20°C.
Under the present invention, the particle size represents a value measured using the aforementioned dynamic light-scattering size distribution measuring system at a measurement temperature of 20°C.

The cosmetic composition proposed by the present invention may be blended, as necessary, with water, polyalcohols, water-soluble polymer compounds, oil-soluble components (oils, waxes), preservatives, antioxidants, aromatic agents, and other substances normally used for cosmetic compositions.

### (Antioxidant)

To maintain stability, preferably antioxidant is blended into the cosmetic composition proposed by the present invention. Any antioxidant can be used without limitation, but examples include polyphenol compounds, radical scavengers, tocopherols and ascorbic acids, among others. Antioxidants that can be used under the present invention include hydrophilic antioxidants and/or oil-soluble antioxidants that may be used alone or combined.
For the aforementioned antioxidant, preferably at least one type of antioxidant selected from the group that includes ascorbic acid and derivatives thereof, and tocopherols and derivatives thereof are contained. Such antioxidant may be added to the water phase or oil phase of the cosmetic composition.
The content of antioxidant in the cosmetic composition is not limited in any way.

### (Ascorbic Acid or Derivative Thereof)

Examples of ascorbic acid or derivative thereof include ascorbic acid, sodium ascorbate, potassium ascorbate, calcium ascorbate, L-ascorbic acid phosphate ester, magnesium ascorbyl phosphate, sodium ascorbyl phosphate, ascorbyl sulfate, sodium ascorbyl 2 phosphate salt and ascorbyl-2-glucoside, and the like. In addition, erythorbic acid or derivative thereof, such as erythorbic acid, sodium erythorbate, potassium erythorbate, calcium erythorbate, erythorbic acid phosphate, erythorbic acid sulfate, etc., is also included in "ascorbic acid or derivative thereof" under the present invention.
Among the above, magnesium ascorbyl phosphate, sodium ascorbyl phosphate, ascorbyl-2-glucoside, and sodium ascorbate are preferable from the viewpoints of preventing fading of carotenoid and stabilizing the dispersion of emulsion particles, of which magnesium ascorbyl phosphate and sodium ascorbyl phosphate are particularly preferable.
For such ascorbic acid or derivative thereof, commercial products may be used as deemed appropriate. Examples include L-ascorbic acids (Takeda Pharmaceutical, Fusou Chemical, BASF Japan, Daiichi Pharmaceutical, etc.), L-ascorbic acid Na (Takeda Pharmaceutical, Fusou Chemical, BASF Japan, Daiichi Pharmaceutical, etc.), ascorbic acid 2-glucoside (brand name: AA-2G: Hayashibara Biochemical Labs), Mg L-ascorbic acid phosphate (brand name: Ascorbyl PM "SDK" (Showa Denko), brand name: NIKKOL VC-PMG (Nikko Chemicals) and brand name: C-Mate (Takeda Pharmaceutical)).

### (Tocopherol or Derivative Thereof)

Any tocopherol can be used without limitation, but it should be selected from the group of compounds that includes tocopherols and derivatives thereof.
Examples of such compound which is tocopherol or derivatives thereof include dl-α-tocopherol, dl-β-tocopherol, dl-γ-tocopherol, dl-δ-tocopherol, acetic acid dl-α-tocopherol, nicotinic acid dl-α-tocopherol,linoleic acid dl-α-tocopherol,succinic acid dl-α-tocopherol, and other tocopherols and derivatives thereof, as well as α-tocotrienol, β-tocotrienol, γ-tocotrienol, δ-tocotrienol, etc. Among these, tocopherols (including dl-α-tocopherol,dl-β-tocopherol, dl-γ-tocopherol, and dl-δ-tocopherol) and tocotrienols (α-tocotrienol, β-tocotrienol, γ-tocotrienol, and δ-tocotrienol) are preferable.
The foregoing are often used as a mixture in a condition called extracted tocopherol or mixed tocopherol.

### (Other Antioxidants)

Polyphenol compounds that can be used as antioxidants include flavonoids (catechin, anthocyanin, flavone, isoflavone, flavan, flavanone and rutin), phenolic acids (chlorogenic acid, ellagic acid, gallic acid and propyl gallate), lignans, curcumins and coumarins, among others. These compounds are contained in large amounts in extracts of the natural substances mentioned below, so they can be used in the form of extracts.

Examples include licorice extract, cucumber extract, Millettia dielsiana extract, Gentiana lutea (Japanese gentian) extract, geranium thunbergii extract, cholesterol or derivative thereof, Chinese hawthorn extract, paeoniae radix extract, ginkgo extract, Scutellaria baicalensis extract, carrot extract, Turkestan rose (Ramanas rose) extract, Cassia mimosoides L. extract, tormentil extract, parsley extract, tree peony (peony root bark) extract, flowering quince (chaenomeles) extract, melissa extract, Alnus firma (cornflower) extract, strawberry begonia extract, rosemary extract, lettuce extract, tea extract (oolong tea, red tea, green tea, etc.), microbial fermentation metabolic products and Siraitia grosvenorii extract, and the like (aliases, galenica names and other names of plants are shown in parentheses). Among these polyphenols, catechin, rosemary extract, glucosyl rutin, ellagic acid, and gallic acid are particularly preferable.

Any commercially available antioxidant can be used under the present invention. Examples include ellagic acid (Wako Pure Chemical Industries, etc.), rosemary extract (brand name: RM-21A, RM-21E: Mitsubishi-Kagaku Foods, etc.), catechin (brand name: Suncatol W-5, No. 1: Taiyo Kagaku, etc.), gallic acid Na (brand name: Suncatol: Taiyo Kagaku, etc.), rutin/glucosyl rutin/enzyme decomposition rutin (brand name: Rutin K-2, P-10: Kiriya Chemical, brand name: αG Rutin: Hayashibara Biochemical Labs, etc.), among others.

### [Oil-soluble Component]

The cosmetic composition proposed by the present invention may contain other oil-soluble components that dissolve in an oil medium.
For such other oil-soluble component, any other component normally used as a UV absorbent, antioxidant, anti-inflammatory agent, moisturizing agent, hair protective agent, dispersant, solvent, whitening agent, anti-pigmentation agent, cell activator, emollient agent, dead skin cell dissolving agent, antistatic agent, vitamins, metabolic syndrome improving agent, anti-hypertensive agent, sedative, etc., may be used. Examples include, among others: olive oil, camellia oil, macadamia nut oil, castor oil and other oils; liquid paraffin, paraffin, Vaseline, ceresin, microcrystalline wax, squalane and other hydrocarbons; carnauba wax, candelilla wax, hohoba oil, honey wax, lanolin and other waxes; isopropyl myristate, 2-octyl dodecyl myristate, 2-cetyl ethyl hexanoate, diisostearyl malate and other esters; palmitic acid, stearic acid, isostearic acid and other fatty acids; cetyl alcohol, stearyl alcohol, isostearyl alcohol, 2-octyl dodecanol and other higher alcohols; methyl polysiloxane, methyl phenyl polysiloxane and other silicone oils; glycerin and other fatty acid esters; and other polymers, oil-soluble coloring agents and oil-soluble proteins. Various plant extract oils and animal extract oils constituted by a mixture of the foregoing are also included. Other preferable oil-soluble components used under the present invention include vitamin Es (other than the aforementioned tocopherols and derivatives thereof), coenzyme Qs and ω-3 oils (EPA, DHA, linoleic acid and other oils), among others.

### (Polyalcohol)

Preferably the cosmetic composition proposed by the present invention contains polyalcohol to demonstrate moisture-retaining function, viscosity-adjustment function, etc. Furthermore, adding polyalcohol lowers the moisture activity of the cosmetic composition and thereby suppresses breeding of microorganisms.

Any polyalcohol can be used under the present invention, as long as it is dihydric alcohol or higher.
Examples of such polyalcohol include, among others, glycerin, diglycerin, triglycerin, polyglycerin, 3-methyl-1,3-butanediol, 1,3-butylene glycol, isoprene glycol, polyethylene glycol, 1,2-pentane diol, 1,2-hexane diol, propylene glycol, dipropylene glycol, polypropylene glycol, ethylene glycol, diethylene glycol, pentaerythritol, neopentyl glycol, maltitol, reduced starch syrup, fruit sugar, grape sugar, sucrose, lactitol, palatinit, erythritol, sorbitol, mannitol, xylitol, xylose, glucose, lactose, mannose, martose, galactose, fructose, inositol, pentaerythritol, maltotriose, sorbitol, sorbitan, trehalose, starch decomposed sugar and starch decomposed sugar reduced alcohol, which may be used alone or as a mixture of multiple types.

### (Water-soluble Polymer Compound)

The water-soluble polymer compound can be selected from a wide selection, where any synthetic polymer, natural polymer or semi-synthetic polymer may be used. In particular, sugar, protein, or complex thereof is preferred.

Examples of the sugar include, but are not limited to, monosaccharides, disaccharides, oligosaccharides, polysaccharides, dextrin, starch derivatives, gums, mucopolysaccharides, and celluloses.
Among these, representative examples include, but are not limited to, agarose, arabinose, amirose, amilopectin, acacia gum, Arabia gum, arabino galactan, alkyl glycoside, alginic acid, sodium alginate, propylene glycol alginate ester, aldose, inulin, oligosaccharide, ghatti gum, curdlan, carageenan, galactomannan, galactose, xanthan gum, xylose, xyloglucan, chitin, chitosan, guar gum, cluster dextrin, β-glucan, glucuronic acid, glycogen, glycosaminoglycan, glycel aldehyde, glucosamine, glucose, glucomannan, ketose, chondroitin sulfuric acid, psyllium seed gum, gellan gum, cyclodextrin, sucrose, hydroxy ethyl cellulose, hydroxy propyl cellulose, carboxy methyl cellulose, methyl cellulose, cellobiose, sorbitol, deoxyribose, dextrin, invert sugar, starch, soybean polysaccharides, sugar alcohol, glycoprotein, tragacanth gum, trehalose, hyaluronic acid, fucose, fructose, pullulan, pectin, heparin, hemicellulose, maltose, mannitol, mannan, lactose, and ribose.
Among these sugars, gums and polysaccharides are preferred as they can achieve stable dispersion due to higher viscosity, and xanthan gum, Arabia gum, pullulan, etc., are more preferable in view of stability of carotenoids.

For the protein, any type of protein can be used as long as it is a polymer or oligomer whose amino acid is peptide-bonded, but natural and water-soluble protein is more preferred.
Proteins are classified into simple proteins constituted by amino acid, and complex proteins containing constituents other than amino acid, and both types of proteins can be used. Examples of simple proteins include gelatin, casein, fibroin, sericin, keratin, protamin, etc. Examples of complex proteins include glycoprotein which is a carbohydrate-bonded form of protein, lipoprotein which is a lipid-bonded form of protein, metal protein which is a metal-ion-bonded form of protein, nucleoprotein which is a ribonucleic acid-bonded form of protein, and phosphoprotein which is a phosphoric-acid-group bonded form of protein.
On the other hand, proteins are generally named after their material, examples of which include animal muscle protein, milk protein, egg protein, rice protein, flour protein (flour gluten), soybean protein, yeast protein and bacterial protein, among others.
These proteins may be used as a mixture.

### (Amino Acid or Derivative Thereof)

The cosmetic composition proposed by the present invention may contain amino acid or derivative thereof. Any amino acid or derivative thereof may be used without limitation, as long as it can be used as a constituent of cosmetic material.
Examples of the amino acid or derivative thereof include glycin, alanine, valine, leucine, isoleucine, serine, threonine, aspartic acid, glutamic acid, cystine, methionine, lysine, hydroxy lysine, alginine, histidine, phenyl alanine, tyrosine, tryptophane, proline, hydroxy proline, acetyl hydroxy proline and other amino acids and derivatives thereof, among others.
Among these amino acids or derivatives thereof, hydroxy proline and acetyl hydroxy proline are preferred.
Such amino acid or derivative thereof may be synthesized using a known method, or a commercial product may be used. These may be used alone or multiple types may be combined.
The content of such amino acid or derivative thereof is not specifically limited, but it is preferably 0.00001 to 10 percent by mass, or more preferably 0.0001 to 5 percent by mass, or yet more preferably 0.0005 to 1 percent by mass, relative to the total mass of the cosmetic composition.

The cosmetic composition proposed by the present invention can also contain aromatic agents.
Any aromatic agent, such as natural aromatic agent of animal type, plant type or mineral type or synthetic aromatic agent, can be used under the present invention, where examples include rose extract, chamomile extract, green tea aromatic agent, lavender oil, geranium oil, jasmine oil, bergamot oil, musk oil, ylang ylang oil, limonene, linalol, citral, cyclopentadecanone, eugenol, and rose oxide, among others.

The cosmetic composition proposed by the present invention may sometimes fade in color due to light, heat, etc. To prevent fading of color, sequestering agents or other compounds having sequestering effect can be blended into the cosmetic material, in addition to the constituents of the cosmetic material, as it improves the stability of color of metal phthalocyanine derivative.
As for sequestering agents and compounds having sequestering effect that can be used under the present invention, sodium EDTA-2, sodium EDTA-3, sodium EDTA-4, etidronic acid, sodium etidronate 4 and hydroxy ethane diphosphonic acid solution, citric acid, succinic acid, phytic acid, ascorbic acid, gluconic acid, phosphoric acid, sodium polyphosphate, and sodium metaphosphate are effective in improving the aforementioned color stability, where phytic acid offers particularly higher color-stability improving effect and is highly effective in preventing the fading of color of iron phthalocyanine derivative. Such sequestering agent or other compound having sequestering effect demonstrates anti-fading effect when contained by 0.01 to 1 percent by mass, or preferably by 0.05 to 0.5 percent by mass, relative to the mass of the cosmetic composition.

### Examples

The present invention is explained using examples below, but it should be noted that the present invention is not at all limited to these examples. Unless otherwise specified, "%" figures in the following text are based on percentage by mass.

### Optimal Concentration Confirmation Test of Iron Phthalocyanine Tetracarboxylic Acid

### (1) Preparation of beauty essences tested

To confirm the optimal concentration of beauty essence containing iron phthalocyanine tetracarboxylic acid, beauty essences were prepared according to a normal method at various concentrations (0.001% in Example 1, 0.003% in Example 2, 0.005% in Example 3, 0.01% in Example 4, 0.02% in Example 5, 0.05% in Example 6, 0.1% in Example 7, 0.2% in Example 8, 0.5% in Example 9). The blending recipes are shown in Table I below.

**[Table 1]**

| Recipe composition | Example 1 | Example 2 | Example 3 | Example 4 | Example 5 | Example 6 | Example 7 | Example 8 | Example 9 |
|---|---|---|---|---|---|---|---|---|---|
| Dipropylene glycol | 4 | 4 | 4 | 4 | 4 | 4 | 4 | 4 | 4 |
| Glycerin | 4 | 4 | 4 | 4 | 4 | 4 | 4 | 4 | 4 |
| Carboxyvinyl polymer | 0.3 | 0.3 | 0.3 | 0.3 | 0.3 | 0.3 | 0.3 | 0.3 | 0.3 |
| PEG-75 | 1 | 1 | 1 | 1 | 1 | I | 1 | 1 | 1 |
| (Acryloyl dimethyl taurine ammonium/VP ) copolymer | 0.2 | 0.2 | 0.2 | 0.2 | 0.2 | 0.2 | 0.2 | 0.2 | 0.2 |
| PEG-80 hydrated caster oil | 0.2 | 0.2 | 0.2 | 0.2 | 0.2 | 0.2 | 0.2 | 0.2 | 0.2 |
| phenoxy ethanol | 0.2 | 0.2 | 0.2 | 0.2 | 0.2 | 0.2 | 0.2 | 0.2 | 0.2 |
| Aromatic agent | 0.01 | 0.01 | 0.01 | 0.01 | 0.01 | 0.01 | 0.01 | 0.01 | 0.01 |
| Iron phthalocyanine Tetracarboxylic acid | 0.001 | 0.003 | 0.005 | 0.01 | 0.02 | 0.05 | 0.1 | 0.2 | 0.5 |
| Potassium hydroxide | 0.11 | 0.11 | 0.11 | 0.11 | 0.11 | 0.11 | 0.11 | 0.11 | 0.11 |
| Purified water | 89.979 | 89.977 | 89.975 | 89.97 | 89.96 | 89.93 | 89.88 | 89.78 | 89.48 |

### [Evaluation Test]

Beauty essence containing iron phthalocyanine tetracarboxylic acid at each concentration was tested on a group of 10 subjects, and after use the subjects were asked to evaluate the feeling of use and effect in a survey.
The test method is as follows:
(1) Subjects: Healthy adult females were selected as subjects, where those having extremely sensitive skin, atopic dermatitis, or skin diseases in areas where cosmetic materials are to be used were excluded.
(2) Cosmetic materials tested: The subjects used the cosmetic materials given in the Examples. The cosmetic materials tested were identified only by a code to protect the details of the Examples from the subjects. A base ensuring sufficient safety and stability was used for the cosmetic materials tested.
(3) Method of use: The subjects were instructed to stop using the skin milk, cream and/or beauty essence they were regularly using, and instead use the cosmetic materials being tested. The subjects were allowed to use other types of cosmetic materials as usual, such as face wash, during the monitor test.
(4) Locations of use: The subjects were instructed to continuously use the cosmetic materials distributed in place of their regular skin milk, cream and/or beauty essence, in similar locations.
(5) Period of use: The subjects were instructed to use the cosmetic materials twice a day (morning and night) for 10 consecutive days.
(6) Amount used: The subjects used the same amount in one application as the amount they would use for their regular cosmetic material.
(7) Evaluation:
   [Anti-aging Effect]
      The subjects judged the actual feeling of use based on a scale of five as specified below:
      1 Not effective
      2 Hardly effective
      3 Slightly effective
      4 Effective
      5 Very effective

      As for the overall evaluation, the cosmetics test received a score of " 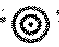 " when at least eight out of the 10 subjects said "Slightly effective," "Effective" or "Very effective," "○" when at least six subjects said the same, "Δ" when four or five subjects said the same, or "×" when no more than three subjects said the same.
   [Appearance]
      Two expert evaluators visually evaluated the appearance of each beauty essence tested and gave an overall judgment, as follows:
      × Inappropriate as a cosmetic
      Δ The cosmetic appears slightly inappropriate, presenting problems
      ○ The cosmetic appears appropriate and good, presenting no problems
      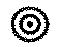 The cosmetic appears excellent

### [Test Results]

Table 2 shows the anti-skin-aging effects and appearance evaluation results, based on an after-use survey, of the beauty essences containing iron phthalocyanine tetracarboxylic acid conforming to the present invention.
Among the beauty essences containing the iron phthalocyanine tetracarboxylic acid of each recipe, anti-skin-aging effect was felt by 60% of subjects when the blending concentration was 0.001% or 0.003%, while the effect was felt by at least 80% of subjects when the blending concentration was 0.005% or more.
In other words, sufficient effect was felt from the tested beauty essences of 0.001 to 0.5% in iron phthalocyanine tetracarboxylic acid content. On the other hand, appearance evaluation found that the beauty essences containing iron phthalocyanine tetracarboxylic acid by 0.1% or less appeared appropriate, presenting no problems. However, those containing iron phthalocyanine tetracarboxylic acid by 0.2% or more had a color that appeared inappropriate as a cosmetic.

**[Table 2]**

| Concentration Dependence of Anti-skin-aging Effect and Appearance of Beauty Essences Containing Iron Phthalocyanine Tetracarboxylic Acid | | | | | | | | | |
|---|---|---|---|---|---|---|---|---|---|
| | Example 1 | Example 2 | Example 3 | Example 4 | Example 5 | Example 6 | Example 7 | Example 8 | Example 9 |
| Blended content of iron phthalocyanine tetracarboxylic acid (%) | 0.001 | 0.003 | 0.005 | 0.01 | 0.02 | 0.05 | 0.1 | 0.2 | 0.5 |
| Anti-skin-aging effect | ○ | ○ | 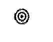 | 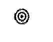 | 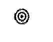 | 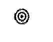 | 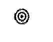 | 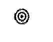 | 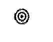 |
| Appearance evaluation | 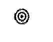 | 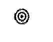 | 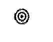 | 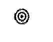 | ○ | ○ | ○ | Δ | Δ |

### Comparison Test against Recipe Not Containing Iron Phthalocyanine Tetracarboxylic Acid

### (1) Preparation of beauty essence tested

Beauty essence containing iron phthalocyanine tetracarboxylic acid conforming to the present invention was prepared by a normal method according to the recipe shown in Table 3 below.
Carboxyvinyl polymer was dissolved in a portion of purified water, after which glycerin, dipropylene glycol and other materials were added one by one, with the mixture agitated fully using a homogenizing mixer. The agitated mixture was cooled, and then potassium hydroxide was added to neutralize the acidity, after which an aromatic agent was added, followed by the remaining purified water, to prepare a gel composition.

**[Table 3]**

| Recipe composition | Recipe of Example 4 P | Comparative Example Q |
|---|---|---|
| Dipropylene glycol | 4 | 4 |
| Glycerin | 4 | 4 |
| Carboxyvinyl polymer | 0.3 | 0.3 |
| PEG-75 | 1 | 1 |
| (Acryloyl dimethyl taurine ammonium/VP) copolymer | 0.2 | 0.2 |
| PEG-80 hydrated caster oil | 0.2 | 0.2 |
| Phenoxy ethanol | 0.2 | 0.2 |
| Aromatic agent | 0.01 | 0.01 |
| Iron phthalocyanine Tetracarboxylic acid | 0.01 | - |
| Potassium hydroxide | 0.11 | 0.11 |
| Purified water | 89.97 | 89.98 |

### [Comparative Example]

Beauty essence was obtained in the same manner as in Example 4, except that no iron phthalocyanine tetracarboxylic acid was added.

### [Evaluation Test]

A use test of the beauty essences given by the Example and Comparative Example as obtained above was conducted on 21 subjects, and after use the subjects were asked to evaluate the feeling of use and effect in a survey.
The test method is as follows:
(1) Subjects: Healthy adult females were selected as subjects, where those having extremely sensitive skin, atopic dermatitis, or skin diseases in areas where cosmetic materials are to be used were excluded.
(2) Cosmetic materials tested: The subjects used the two types of cosmetic materials given in the Example and Comparative Example. The cosmetic materials tested were identified only by a code to protect the identity of Example and Comparative Example from the subjects. A base ensuring sufficient safety and stability was used for the cosmetic materials tested, and there was no difference in usage between the two cosmetic materials.
(3) Method of use: The subjects were instructed to stop using the skin milk, cream and/or beauty essence they were regularly using, and instead use the cosmetic materials tested. The subjects were allowed to use other types of cosmetic materials as usual, such as face wash, during the monitor test.
(4) Locations of use: The subjects were instructed to continuously use one of the cosmetic materials distributed on one side of the face, and the other on the other side of the face, in place of their regular skin milk, cream and/or beauty essence.
(5) Period of use: The subjects were instructed to use the cosmetic materials twice a day (morning and night) for 10 consecutive days.
(6) Amount used: The subjects used the same amount in one application as the amount they would use for their regular cosmetic material.
(7) Evaluation: The subjects judged the actual feeling of use based on a scale of five as specified below:
   1 Not effective
   2 Hardly effective
   3 Slightly effective
   4 Effective
   5 Very effective
(8) Survey: The survey questions are shown in the table below together with the test results. Statistical analysis used Excel software for t-test, "test method using two samples assuming unequal variance."

### [Test Results]

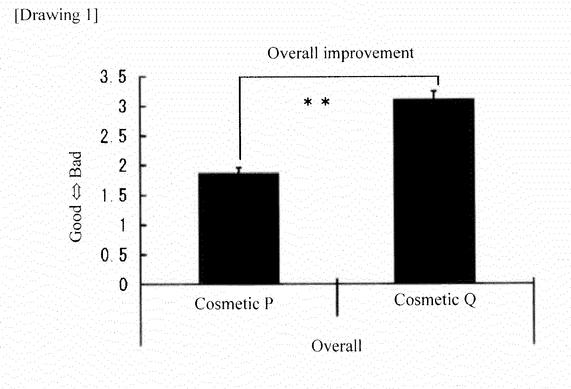

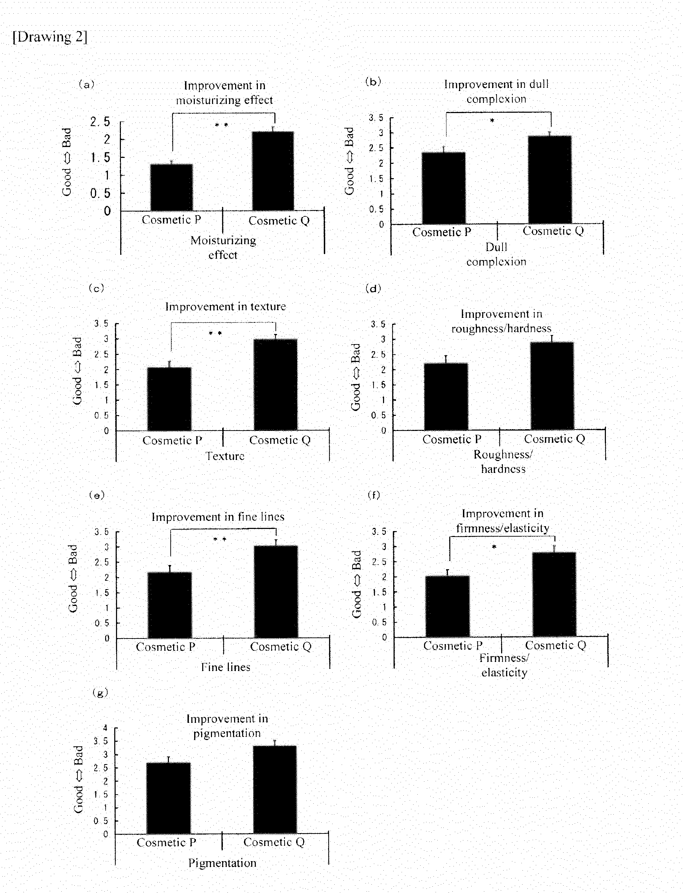

As shown in Table 4 and Figs. 1 and 2 below, the beauty essence containing iron phthalocyanine tetracarboxylic acid conforming to the present invention received significantly higher scores than the beauty essence not containing iron phthalocyanine tetracarboxylic acid in terms of the actual feelings of "moisturizing effect," "improvement in dull complexion," "improvement in texture," "improvement in fine lines," "improvement in firmness/elasticity" and "overall improvement of the skin." In particular, the former rated markedly higher in "moisturizing effect," "improvement in texture" and "overall improvement of the skin" (1% significance).
When asked which of the beauty essences given by the Example and Comparative Example was felt effective in improving the skin, 14 out of 21 subjects said that the beauty essence in the "Example was good," four said that the beauty essence in the "Example was slightly better," and three said that there was "No difference." These results suggest an overwhelmingly higher skin improvement effect of the beauty essence given by the Example.

**[Table 4]**

| | Question | Beauty essence felt more effective | Significant difference |
|---|---|---|---|
| Q1. | Did you feel moisturizing effect? | Example P | ** |
| Q2. | Did you feel improvement in dull complexion? | Example P | * |
| Q3. | Did you feel improvement in texture? | Example P | ** |
| Q4. | Did you feel improvement in roughness/hardness of skin surface? | | |
| Q5. | Did you feel improvement in fine lines? | Example P | ** |
| Q6. | Did you feel improvement in firmness/elasticity? | Example P | * |
| Q7. | Did you feel improvement in pigmentation? | | |
| Q8. | Did you feel overall improvement of the skin? | Example P | ** |

| | | | |
|---|---|---|---|
| *: 5% significance **: 1% significance | | | |

### Effectiveness Test of Beauty Essence Containing Iron Phthalocyanine Octacarboxylic Acid Example 10

### [Preparation of Beauty Essence]

Beauty essence was prepared by a normal method in the same manner as in Example 4 above, except that iron phthalocyanine octacarboxylic acid was used instead of iron phthalocyanine tetracarboxylic acid.

### [Evaluation Test]

A use test of the beauty essence given by Example 10 as obtained above was conducted on 21 subjects, and after use the subjects were asked to evaluate the feeling of use and effect in a survey.
The test was conducted in the same manner as with the aforementioned examples.
The test method is as follows:
(1) Subjects: Healthy adult females were selected as subjects, where those having extremely sensitive skin, atopic dermatitis, or skin diseases in areas where cosmetic materials are to be used were excluded.
(2) Cosmetic material tested: The subjects used the cosmetic material given in the Example. The cosmetic material tested was identified only by a code to protect the details of the Example from the subjects. A base ensuring sufficient safety and stability was used for the cosmetic material tested.
(3) Method of use: The subjects were instructed to stop using the skin milk, cream and/or beauty essence they were regularly using, and instead use the cosmetic material tested. The subjects were allowed to use other types of cosmetic materials as usual, such as face wash, during the monitor test.
(4) Locations of use: The subjects were instructed to continuously use the cosmetic material distributed in place of their regular skin milk, cream and/or beauty essence, in similar locations.
(5) Period of use: Same as above.
(6) Amount used: Same as above.
(7) Evaluation: After using the monitor product, the subjects were instructed to evaluate "improvement in firmness/elasticity," "moisturizing effect," "improvement in fine lines," "improvement in dull complexion," "improvement in roughness/hardness of skin surface," "improvement in texture," "improvement in pigmentation" and "overall improvement of the skin" based on a scale of two as specified below compared to before using the product:
   1 No
   2 Yes

### [Test Results]

The subjects' responses to each of the survey questions are shown below, together with the number of subjects saying "Yes" and their percentage shown in parentheses, obtained after using the beauty essence given by the Example containing iron phthalocyanine octacarboxylic acid conforming to the present invention:
- Did you feel improvement in firmness/elasticity? (Yes: 17 subjects, 81%)
- Did you feel moisturizing effect? (Yes: 17 subjects, 81%)
- Did you feel improvement in fine lines? (Yes: 9 subjects, 43%)
- Did you feel improvement in dull complexion? (Yes: 9 subjects, 43%)
- Did you feel improvement in roughness/hardness of skin surface? (Yes: I I subjects, 52%)
- Did you feel improvement in texture? (Yes: 13 subjects, 62%)
- Did you feel improvement in pigmentation? (Yes: 5 subjects, 24%)
- Did you feel overall improvement of the skin? (Yes: 19 subjects, 91%)
With the beauty essence given by the Example containing iron phthalocyanine octacarboxylic acid conforming to the present invention, the percentages of subjects feeling "improvement in firmness/elasticity" and "moisturizing effect" were markedly high at 80% or above, and there was also a high percentage or over 60% of subjects who felt "improvement in texture". The percentage of subjects feeling "overall improvement of the skin" was markedly high at 91%.

### Example 11

### Use Test of Beauty Essence Containing Iron Phthalocyanine Tetracarboxylic Acid

### [Preparation of Beauty Essence Tested]

Beauty gel containing iron phthalocyanine tetracarboxylic acid by 0.005 percent by mass according to the present invention (Example 11) was prepared by a normal method based on the recipe specified below.

### [Blending Recipe of Iron Phthalocyanine Tetracarboxylic Acid]

10% of glycerin, 6% of 1,3-butylene glycol, 4% of diglycerin, 1% of cyclohexane dicarboxylic acid bis-ethoxy diglycol, 3% of raffinose, 2% of dipropylene glycol, 0.8% of bis-ethoxy diglycol succinate, 1.5% of glucosyl trehalose, 0.5% of polysolvate, 0.4% of carbomer, 0.3% of xanthan gum, 0.01% of hydrolyzed hyaluronic acid, 0.01% of acetyl hyaluronate Na, 0.1% of phytic acid, 0.05% of ascorbyl glucoside, appropriate amount of beauty component, appropriate amount of aromatic component, 0.6% of phenoxy ethanol, 0.005% of iron phthalocyanine tetracarboxylic acid, purified water for the remainder.
The beauty gel obtained by this recipe had good stability.

### [Evaluation Test]

A use test of the beauty gel given in Example 11 as obtained above was conducted on 25 subjects, and after use the subjects were asked to evaluate the feeling of use and effect in a survey. The test method is as follows:
(1) Subjects: Healthy adult females were selected as subjects, where those having extremely sensitive skin, atopic dermatitis, or skin diseases in areas where cosmetic materials are to be used were excluded.
(2) Cosmetic material tested: The subjects used the cosmetic material tested given in the Example. The cosmetic material tested was identified only by a code to protect the identity of the Example from the subjects. A base ensuring sufficient safety and stability was used for the cosmetic materials tested.
(3) Method of use: The subjects were instructed to stop using the skin milk, cream and/or beauty essence they were regularly using, and instead use the cosmetic material tested. The subjects were allowed to use other types of cosmetic materials as usual, such as face wash, during the monitor test.
(4) Locations of use: The subjects were instructed to continuously use the cosmetic material distributed in place of their regular skin milk, cream and/or beauty essence, in similar locations.
(5) Period of use: The subjects were instructed to use the cosmetic material twice a day (morning and night) for 10 consecutive days.
(6) Amount used: The subjects used the same amount in one application as the amount they would use for their regular cosmetic material.
(7) Evaluation: After using the gel, the subjects were instructed to evaluate feeling of "improvement in firmness/elasticity," "glowing complexion," "moisturizing effect," "texture," "roughness/hardness of skin surface," "fine line," "pigmentation" and "overall improvement" based on a scale of five as specified below compared to before using the product. They were also instructed to evaluate the same items for the cosmetics they were using before the start of the test:
   1 No
   2 Not much
   3 Average
   4 Slightly
   5 Yes

### [Test Results]

The number and percentage of subjects saying "Yes" or "Slightly" to each of the questions on feeling after use of the beauty essence containing iron phthalocyanine tetracarboxylic acid according to the recipe of Example 11 as well as feeling of use of the cosmetics they were currently using, are shown in Table 5 below.

**[Table 5]**

| Question | Total 25 subjects | | % | |
|---|---|---|---|---|
| | Current cosmetics | Monitor product | Current cosmetics | Monitor product |
| Improvement in firmness/elasticity | 12 | 19 | 48 | 76 |
| Glowing complexion | 3 | 16 | 12 | 64 |
| Moisturizing effect | 15 | 20 | 60 | 80 |
| Improvement in texture | 4 | 14 | 16 | 56 |
| Improvement in roughness/hardness of skin surface | 2 | 7 | 8 | 28 |
| Improvement in fine lines | 6 | 7 | 24 | 30 |
| Improvement in pigmentation | 4 | 5 | 16 | 20 |
| Overall improvement of the skin | 8 | 16 | 32 | 64 |

Many subjects who used the test product conforming to the present invention felt "improvement in firmness/elasticity," "moisturizing effect," "glowing complexion" and "improvement in texture." For all of "improvement in firmness/elasticity," "glowing complexion" and "improvement in texture," more than a half of the subjects felt the product was effective, but less than a half of them felt their current cosmetics were effective. These results show that the beauty essence conforming to the present invention was felt markedly more effective in improving the skin than commercial cosmetics. In addition, 64% of the subjects using the product conforming to the present invention (16 subjects) said "Yes" or "Slightly" on "overall improvement of the skin," but the same percentage was 32% (8 subjects) for the cosmetics the subjects were using. The beauty essence conforming to the present invention was felt markedly more effective in improving the skin than commercial cosmetics.

### Example 12

### Effectiveness Test on Suppression of Fading of Iron Phthalocyanine Tetracarboxylic Acid

As mentioned earlier, the cosmetic composition proposed by the present invention stabilizes the color of the metal phthalocyanine derivative conforming to the present invention by blending a sequestering agent or other component having sequestering effect in the cosmetic material.

Beauty essences were prepared by a normal method according to the recipe of Example 4 (Table 3) containing 0.01% of iron phthalocyanine tetracarboxylic acid, by further blending 0.05% or 0.1% of phytic acid, or 0.1% or 0.2% of sodium EDTA-2.

### [Evaluation Test]

Each beauty essence was kept for one week at 50°C and its stability was visually evaluated compared to the beauty essence immediately after preparation.
The evaluation standards are as follows:
[Anti-fading Result]
Expert testers judged the beauty essences tested on a scale of three as specified below:
× Significantly faded.
Δ Slightly faded.
○ Hardly faded.

### [Test Results]

Table 6 shows the results of examining the anti-fading effect of beauty essences containing 0.01% of iron phthalocyanine tetracarboxylic acid as well as component having sequestering effect. Marked fading occurred when EDTA-2 Na was blended by 0.1%, and some fading still occurred when it was blended by 0.2%. This indicates that if the recipe contains EDTA-2 Na, it must be added by 0.2 percent by mass relative to the cosmetic composition. On the other hand, hardly any fading was observed when phytic acid was blended by 0.05% or 0.1%, showing a high anti-fading effect of phytic acid.

**[Table 6]**

| Anti-fading component | | Judgment of appearance |
|---|---|---|
| EDTA-2Na | 0.10% | Δ ∼ × |
| | 0.20% | Δ |
| Phytic acid | 0.05% | ○ |
| | 0.10% | ○ |

### [Example of Recipe 1] Skin lotion

3 percent by mass of ethanol, 5% of glycerin, 2% of 1,3-butylene glycol, 3% of POE sorbitan monostearate, 0.005% of iron phthalocyanine tetracarboxylic acid, purified water for the remainder.
Skin lotion was manufactured by a normal method according to the above recipe. The obtained skin lotion had good stability.

### [Example of Recipe 2] Skin lotion

Skin lotion was manufactured by a normal method according to same blending quantities as the recipe for skin lotion in Example of Recipe 1, except that iron phthalocyanine octacarboxylic acid was used instead of iron phthalocyanine tetracarboxylic acid. The obtained skin lotion had good stability.

### [Example of Recipe 3] Skin milk

6 percent by mass of squalane, 1% of cetyl alcohol, 1.5% of olive oil, 1.5% of macadamia nut oil, 3% of glycerin, 3% of 1,3-butylene glycol, 0.5% of carboxyvinyl polymer, 0.15% of sodium hydroxide, 2% of decaglyceryl monostearate, 0.01% of iron phthalocyanine tetracarboxylic acid, appropriate amount of aromatic agent, 0.8% of phenoxy ethanol, purified water for the remainder.
Skin milk was manufactured by a normal method according to the above recipe. The obtained skin milk had good stability.

### [Example of Recipe 4] Cream

10 percent by mass of squalane, 5% of octyl dodecanol, 30% of glycerol tri-2-ethyl hexaonate ester, 3% of honey wax, 2% of Vaseline, 4% of 1,3-butylene glycol, 5% of propylene glycol monostearate, 0.01% of iron phthalocyanine tetracarboxylic acid, appropriate amount of aromatic agent, 0.8% of phenoxy ethanol, purified water for the remainder.
Cream was manufactured by a normal method according to the above recipe. The obtained cream had good stability.

### [Example of Recipe 5] Cream

Cream was manufactured by a normal method according to same blending quantities as the recipe for cream in Example of Recipe 4, except that iron phthalocyanine octacarboxylic acid was used instead of iron phthalocyanine tetracarboxylic acid. The obtained cream had good stability.

### [Example of Recipe 6] Bath agent

50 percent by mass of sodium sulfate, 49.98% of sodium hydrogen carbonate, 0.01% of iron phthalocyanine tetracarboxylic acid, 0.01% of aromatic agent.
Bath agent was manufactured by a normal method according to the above recipe. The obtained bath agent had good stability.

### [Example of Recipe 7] Shampoo

40 percent by mass of glycerin, 12% of N-palm oil fatty acid acyl-L-potassium glutamate, 3% of N-lauroyl-β-sodium methyl alanine, 10% of 2-alkyl-N-carboxy methyl-N-hydroxy ethyl imidazolinium betaine, 1% of diethanol amide laurate, 5% of ethanol, 1% of sodium carboxy methyl cellulose, 0.01% of iron phthalocyanine tetracarboxylic acid, appropriate amount of aromatic agent, 0.9% of phenoxy ethanol, purified water for the remainder.
Shampoo was manufactured by a normal method according to the above recipe. The obtained shampoo had good stability.

### [Example of Recipe 8] Body soap

2% of 1,2-pentane diol, 15% of diglycerin, 10% of maltitol, 15% of N-lauroyl-L-sodium glutamate, 1% of polyoxy ethylene (20EO) sorbitan monolaurate, 1% of palm oil fatty acid polyoxy ethylene (7EO) glyceryl, 12% of ethanol, 1% of sodium carboxy methyl cellulose, 0.01% of iron phthalocyanine tetracarboxylic acid, appropriate amount of aromatic agent, 0.9% of phenoxy ethanol, purified water for the remainder.
Body soap was manufactured by a normal method according to the above recipe. The obtained body soap had good stability.

### Industrial Field of Application

The cosmetic composition proposed by the present invention can be applied to beauty essence (water-based or gel type), skin lotion, skin milk, cream, foundation, shampoo, body soap, bath agent, and the like.

## Claims

1. A cosmetic composition **characterized by** containing a metal phthalocyanine derivative expressed by General Formula [I] below: 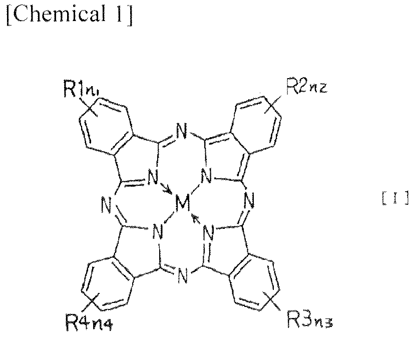 (in Formula [I], M represents a metal selected from the group that includes Fe, Co, Mn, Ti, V, Ni, Cu, Zn, Mo, W and Os, R1 to R4 are each a carboxylic group, and n1 to n4 are each an integer of 0 to 4 satisfying "1 ≤ n1 + n2 + n3 + n4 ≤ 8").

2. A cosmetic composition according to Claim 1, **characterized by** containing, as an effective ingredient, a metal phthalocyanine derivative per Formula [I] above where R1 to R4 are each a carboxylic group and n 1 to n4 all take the same value of 1 or 2.

3. A cosmetic composition according to Claim I or 2, **characterized by** containing, as an effective ingredient, a metal phthalocyanine derivative per Formula [I] above where M is Fe, Co or Mn.

4. A cosmetic composition according to any one of Claims 1 to 3, wherein the cosmetic composition is used for skincare cosmetic materials.

5. A cosmetic composition according to any one of Claims 1 to 4, wherein the metal phthalocyanine derivative is an iron phthalocyanine tetracarboxylic acid or salt thereof.

6. A cosmetic composition according to any one of Claims I to 4, wherein the metal phthalocyanine derivative is an iron phthalocyanine octacarboxylic acid or salt thereof.

7. A cosmetic composition **characterized by** containing a metal phthalocyanine derivative expressed by Formula [I] above as well as a sequestering agent or a component having sequestering effect.

8. A cosmetic composition according to Claim 7, wherein the sequestering agent is at least one type of substance selected from the group that includes sodium EDTA-2, sodium EDTA-3, sodium EDTA-4, etidronic acid, sodium etidronate 4, hydroxy ethane diphosphonic acid solution, and citric acid, while the component having sequestering effect is at least one type of substance selected from the group that includes succinic acid, phytic acid, ascorbic acid, gluconic acid, phosphoric acid, sodium polyphosphate, and sodium metaphosphate.

9. A cosmetic composition according to Claim 7 or 8, **characterized by** containing, as an effective ingredient, a metal phthalocyanine derivative per Formula [I] above where R1 to R4 are each a carboxylic group and n1 to n4 all take the same value of 1 or 2.

10. A cosmetic composition according to any one of Claims 7 to 9, **characterized by** containing, as an effective ingredient, a metal phthalocyanine derivative per Formula [I] above where M is Fe, Co or Mn.

11. A cosmetic composition according to any one of Claims 7 to 10, wherein the cosmetic composition is used for skincare cosmetic materials.

12. A cosmetic composition according to any one of Claims 7 to 11, wherein the metal phthalocyanine derivative is an iron phthalocyanine tetracarboxylic acid or salt thereof.

13. A cosmetic composition according to any one of Claims 7 to 11, wherein the metal phthalocyanine derivative is an iron phthalocyanine octacarboxylic acid or salt thereof.
